(19) Europäisches Patentamt
European Patent Office
Office européen des brevets

(11) **EP 2 127 591 A1**

(12) **EUROPEAN PATENT APPLICATION**
published in accordance with Art. 153(4) EPC

(43) Date of publication:
**02.12.2009 Bulletin 2009/49**

(51) Int Cl.:
**A61B 1/00** (2006.01)

(21) Application number: **07850727.4**

(22) Date of filing: **17.12.2007**

(86) International application number:
**PCT/JP2007/074239**

(87) International publication number:
**WO 2008/120422 (09.10.2008 Gazette 2008/41)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **29.03.2007 JP 2007086484**

(71) Applicant: **Olympus Medical Systems Corp.
Tokyo 151-0072 (JP)**

(72) Inventors:
• **NAITO, Kimihiko
Hachioji-shi, Tokyo 192-8512 (JP)**

• **ISHIGURO, Tsutomu
Hachioji-shi, Tokyo 192-8512 (JP)**
• **HASEGAWA, Jun
Hachioji-shi, Tokyo 192-8512 (JP)**
• **NAKAMURA, Toshio
Hachioji-shi, Tokyo 192-8512 (JP)**

(74) Representative: **von Hellfeld, Axel
Wuesthoff & Wuesthoff
Patent- und Rechtsanwälte
Schweigerstrasse 2
81541 München (DE)**

(54) **ARTICULATED BENDING MECHANISM AND ARTICULATED MEDICALDEVICE WITH ARTICULATED BENDING MECHANISM**

(57)     In a first bending piece (51), a second bending piece (52), and a third bending piece (53) disposed sequentially from an extreme end side and manipulation wires (56a, 56b) for rotating the first bending piece (51), manipulation wires (57a, 57b) for rotating the second bending piece (52), and manipulation wires (58a, 58b) for rotating the third bending piece (53), the manipulation wires (56a, 56b) and the manipulation wires (57a, 57b) are disposed concentrically with respect to the direction of a center axis L.

FIG. 5C

**Description**

Technical Field

The present invention relates to a multijointed bending mechanism in which a plurality of bending pieces can be independently manipulated by manipulation wires, and to a multijointed medical equipment having such multijointed bending mechanism.

Background Art

In general, an insertion portion of a piece of medical equipment such as an endoscope is provided with a bending portion. Bending pieces are rotatably coupled with each other in the bending portion. A manipulation wire is connected only to a bending piece at the most extreme end of the bending portion. The bending portion is bent in its entirety by pushing and pulling the manipulation wire. More specifically, since the respective bending pieces cannot be independently rotated, it is difficult for the bending pieces to take a predetermined bending state.
To cope with the above problem, in Patent Document 1, repellent force application means is disposed in the base end portion of a bending portion. When the bending portion is bent by a manipulation wire, the repellent force application means begins to bend the bending portion preferentially from the extreme end portion thereof. Further, in Patent Document 2, balloons are interposed between bending pieces, respectively. Rotation intervals between bending pieces are adjusted by the expansion and contraction of the balloons. With this configuration, when a bending portion is bent, the radius of bending of the bending portion can be variably adjusted.

Patent Document 1: Jpn. Pat. Appln. KOKAI Publication No. 2003-126024

Patent Document 2: Jpn. Pat. Appln. KOKAI Publication No. 06-105797

Disclosure of Invention

In a conventional bending mechanism, when a manipulation wire is pulled and a bending portion is bent, all the bending pieces are rotated in the same direction. Accordingly, there is a possibility that the bending pieces cannot be independently rotated in an arbitrary direction. As a result, since the bending mode of the bending portion is limited, it may be impossible to rotate only a particular part of the bending portion in a desired direction and thus there is a possibility that the degree of freedom of a bending mode is reduced.
When the bending state of the bending portion is regulated by the repellent force application means as disclosed in Patent Document 1 and by balloons as disclosed in Patent Document 2, only a part of the bending portion is preferentially bent. Accordingly, since it is impossible to selectively rotate an arbitrary bending piece, there is a possibility that a desired bending state cannot be attained. When manipulation wires are disposed in respective bending pieces to increase the degree of freedom of the bending state, the number of the manipulation wires is increased. Thus, the manipulation wires are entangled with each other, and the inside of the bending portion is liable to become complicated.
Accordingly, the present invention provides a multijointed bending mechanism, in which only an arbitrary bending pieces can be independently rotated and manipulation wires can be disposed compactly without being entangled with each other, and multijointed medical equipment having the multijointed bending mechanism.
According to one aspect of the present invention, there is provided a multijointed bending mechanism comprising: a first bending piece; a second bending piece connected to the first bending piece so as to be rotatable around a first rotation shaft; a third bending piece connected to the second bending piece so as to be rotatable around a second rotation shaft; at least two first wires connected to the first bending piece to rotate the first bending piece; and at least two second wires connected to the second bending piece to rotate the second bending piece, wherein the first wires and the second wires are disposed concentrically with respect to a vertical direction of the first rotation shaft and the second rotation shaft.
According to one aspect of the present invention, there is provided a multijointed bending mechanism comprising: a first bending piece; a second bending piece connected to the first bending piece so as to be rotatable around a first rotation shaft; a third bending piece connected to the second bending piece so as to be rotatable around a second rotation shaft; a fourth bending piece connected to the third bending piece so as to be rotatable around a third rotation shaft; at least two first wires connected to the first bending piece to rotate the first bending piece; at least two second wires connected to the second bending piece to rotate the second bending piece; and at least two third wires connected to the third bending piece to rotate the third bending piece, wherein the first wires, the second wires, and the third wires are concentrically disposed in a vertical direction of the first rotation shaft, the second rotation shaft, and the third rotation shaft.

An aspect of the present invention provides a multijointed medical equipment having the multijointed bending mechanism.

Brief Description of Drawings

FIG. 1 is a perspective view schematically showing an endoscope apparatus included in an endoscope system according to an embodiment of the present invention.

FIG. 2 is a perspective view schematically showing an endoscope and a surgical instrument in the endoscope system according to the embodiment.

FIG. 3 is a perspective view schematically showing the surgical instrument according to the embodiment.

FIG. 4 is a perspective view showing an extreme end portion and a bending portion in an insertion portion of the surgical instrument according to the embodiment.

FIG. 5A is a horizontal longitudinal sectional view of the bending portion along line A-A in FIG. 4 in the long axis direction of the insertion portion, as viewed from above.

FIG. 5B is a vertical longitudinal sectional view of the bending portion along line B-B in FIG. 4 in the long axis direction of the insertion portion, as viewed from the left side thereof.

FIG. 5C is a sectional view taken along an arrow line A-A in FIG. 5A.

FIG. 5D is a perspective view of a separator.

FIG. 6A shows the angular relationship under which bending pieces rotate and is a longitudinal sectional view of the bending portion from above.

FIG. 6B shows the angular relationship under which the bending pieces rotate and is a longitudinal sectional view of the bending portion as viewed from above.

FIG. 7A is an explanatory view of a multijointed structure in the bending portion of a surgical instrument.

FIG. 7B is an explanatory view of the multijointed structure in the bending portion of the surgical instrument.

FIG. 8 is an explanatory view of a multijointed structure in a joystick.

FIG. 9 is a view showing other modification of positioning/disposing means in the embodiment.

FIG. 10 is a view showing other modification of the positioning/disposing means in the embodiment.

FIG. 11 is a view showing other modification of the positioning/disposing means in the embodiment.

FIG. 12 is an explanatory view of a multijointed structure in a bending portion of a surgical instrument in another embodiment of the present invention.

FIG. 13 is an explanatory view of a multijointed structure of a joystick in the embodiment.

Best Mode for Carrying Out the Invention

A multijointed surgical instrument (for example, multijointed medical equipment) having a multijointed bending mechanism according to an embodiment of the present invention and an endoscope system having such multijointed surgical instrument will be explained below with reference to the drawings.

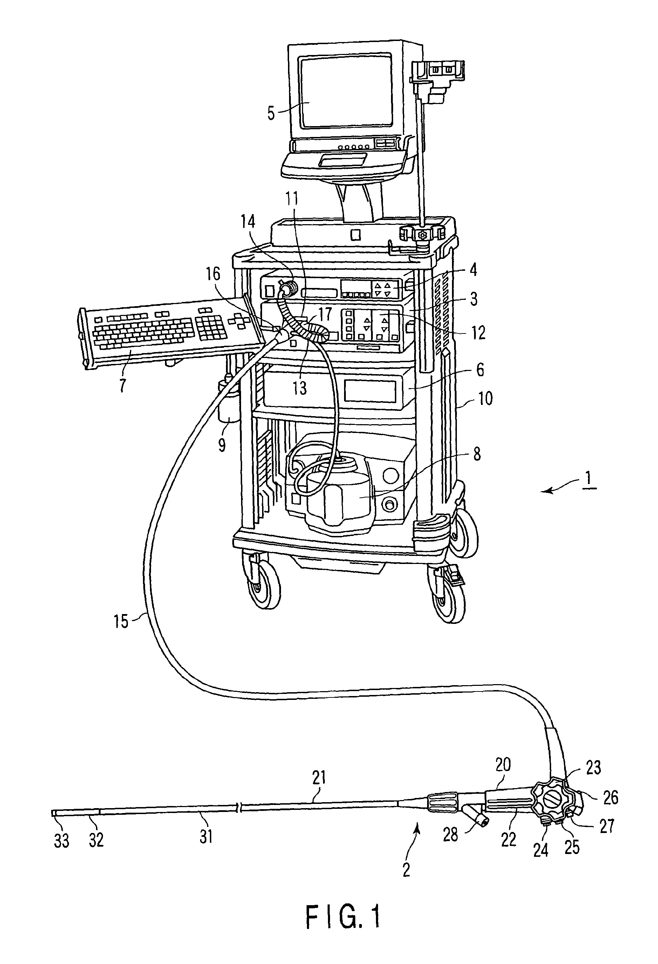

FIG. 1 is a perspective view schematically showing an endoscope apparatus 1 included in the endoscope system. The endoscope apparatus 1 is composed of an electronic endoscope (endoscope main body) 2 and a peripheral device (device main body) of the endoscope 2.

The peripheral device includes a light source unit 3 for creating endoscope illumination light, an image processing unit 4 for subjecting an image picked up by an image pickup portion (not shown) in the endoscope 2 to various types of image processing, an image display unit (for example, monitor) 5 for displaying an image, image data (the image processed by the image processing unit 4), a state of the device, an instruction of an operator, and the like, a controller 6 for overall control of the endoscope system and executing an arithmetic operation and the like, an input unit 7 having a keyboard and the like, a waste fluid tank 8 with a suction pump, a water feed tank 9, and the like. The peripheral device is mounted on a trolley 10.

The light source unit 3 has a connection port 11 connected to a connector unit 16 and a display 12 for displaying an operating state of the light source unit 3 on the front surface thereof.

The image processing unit 4 has a connector receiver 14 connected to a connection cable 13 on a front surface thereof. A connecting unit 17 with a cap is disposed in the base end of the connection cable 13. Further, the connector unit 16 is disposed in the extreme end of a universal cord 15 of the endoscope 2. An electrical connection portion of the connector unit 16 is detachably connected to the connecting unit 17 with the cap.

An image pickup signal obtained in the image pickup portion is sent to the image processing unit 4 through the connection cable 13 and converted to a video signal. The video signal is displayed on the image display unit 5 as an image picked up by the endoscope 2.

Although the endoscope 2 is an electronic endoscope for picking up an endoscope image by an image pickup portion (not shown image pick-up device) disposed in the extreme end of a later-described insertion portion 21, it may be, for example, a fiber endoscope using an image guide fiber. When the fiber endoscope is used, an optical image guided by the image guide fiber is picked up by a TV camera or the like.

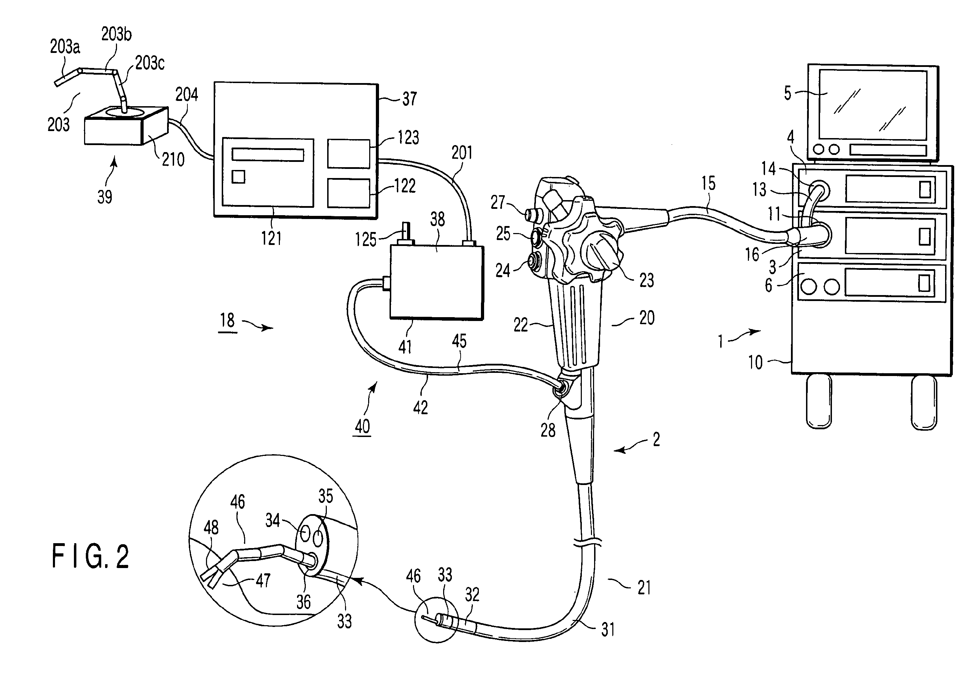

As shown in FIGS. 1 and 2, the endoscope 2 has a manipulation portion 20 and the insertion portion 21 as a base member.

The universal cord 15 is connected to the manipulation portion 20. A grip portion 22 is disposed in the manipulation portion 20. The manipulation portion 20 is provided with various types of function manipulation members, such as an angle manipulation knob 23, an air/water feed manipulation button 24, a suction manipulation button 25, a gas supply manipulation button 26, and switches 27. The function manipulation members are disposed in portions nearer to a proximal end side than the position of the grip portion 22. Further, an insertion port 28 of an insertion channel, into which a later-described surgical instrument 40 and the like are inserted, is disposed in a portion which is positioned nearer to an extreme end side than the position of the grip portion 22.

As shown in FIGS. 1 and 2, the insertion portion 21 is composed of a flexible tube (soft portion) 31 positioned to the proximal end side, a bending portion 32 connecting to the extreme end of the flexible tube 31, and an extreme end portion 33 connected to the extreme end of the bending portion 32. The flexible tube 31 has elasticity and flexibility and is bent by an external force. The bending portion 32 is forcibly bent by manipulating the angle manipulation knob 23. The position and the direction of the extreme end portion 33 are changed by bending the bending portion 32 so that a desired observation target (affected area and the like) is captured in an observation field of view (or in an image pickup field of view).

As shown in FIG. 2, an observation window 34, an illumination window 35, and a channel port 36 are disposed in the extreme end surface portion of the extreme end portion 33.

An image pickup unit, which includes an optical system composed of an objective lens (not shown) and the like and an image pick-up device such as a CCD, is disposed inside the observation window 34. The image pick-up unit picks up an affected area and the like in a body cavity. An image pick-up signal obtained by the image pick-up unit is sent to the image processing unit 4 through the connection cable 13 as described above.

The channel port 36 communicates with the insertion port 28 through an insertion channel (not shown) formed in the insertion portion 21. The insertion channel is used as a path through which an insertion portion 42 of a multijointed surgical instrument 40 for an endoscope is inserted.

Although it is assumed in the embodiment that one surgical instrument 40 is inserted into one insertion channel, a plurality of surgical instruments 40 may be inserted into the one insertion channel. Further, it is also possible to provide a plurality of the insertion channels and to insert each of the surgical instruments 40 into each of the insertion channels.

Next, a surgical instrument extreme end movement controller 18 will be explained with reference to FIGS. 2, 3, 4, 5A and 5B. As shown in FIG. 2, the surgical instrument extreme end movement controller 18 includes a surgical instrument controller 37, a surgical instrument drive unit (motor unit) 38, a bending manipulation unit (manipulation input unit) 39, and the surgical instrument 40.

The surgical instrument 40 includes a manipulation unit 41 which can be gripped by an operator and the insertion portion 42 coupled with the manipulation unit 41.

The surgical instrument drive unit 38 is assembled to the manipulation unit 41.

As shown in FIG. 2, the insertion portion 42 is inserted into a body cavity through the insertion channel. The insertion portion 42 is composed of a flexible tube (soft portion) 45 which is positioned on the proximal end (base end) side, a bending portion 46 connected to the extreme end of the flexible tube 45, and an extreme end portion 47 connected to the extreme end of the bending portion 46.

The flexible tube 45 has elasticity and flexibility and is bent by an external force.

The bending portion 46 is bent by the manipulation unit 41.

The extreme end portion 47 is provided with a grip forceps 48 as a surgical instrument for operating on an affected area and the like.

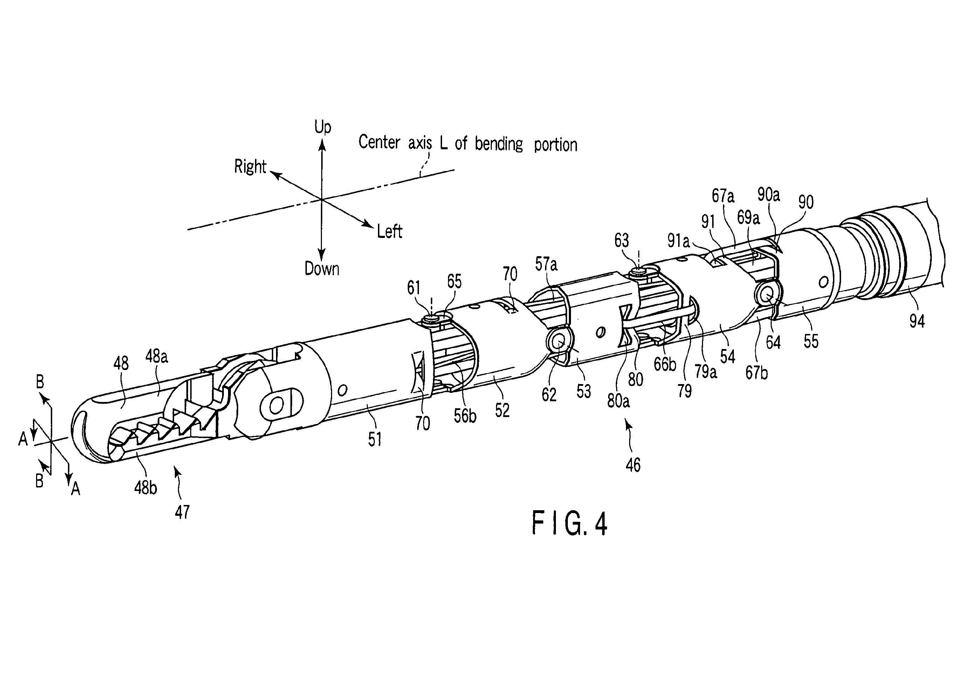

As shown in FIG. 4, the grip forceps 48 includes grip members 48a, 48b which are opened and closed up and down. The grip members 48a, 48b are opened and closed in up and down directions by a manipulation wire 92 inserted into the insertion portion 42. The extreme end portion 47 may be provided with a surgical instrument such as a high-frequency knife or a high-frequency solidifier in addition to the grip forceps 48.

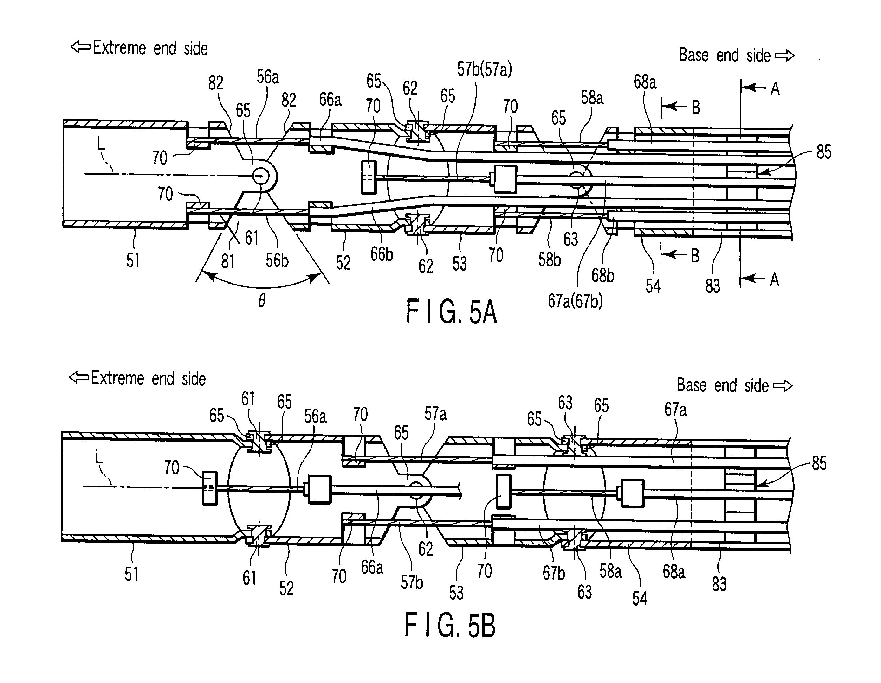

As shown in FIGS. 4, 5A and 5B, the bending portion 46 includes the multijointed bending mechanism. The multijointed mechanism is constructed by coupling bending pieces 51, 52, 53, 54. FIG. 4 is a perspective view showing the extreme end portion 47 and the bending portion 46. FIG. 5A is a horizontal longitudinal sectional view of the bending portion 46 along line A-A in FIG. 4 in the long axis direction of the insertion portion 42 as viewed from above. FIG. 5B is a vertical longitudinal sectional view of the bending portion 46 along line B-B in FIG. 4 in the long axis direction of the insertion portion 42 as viewed from the left side thereof. The up, down, right, and left directions of the bending portion 46 are as shown by indexes of FIG. 4.

The bending pieces 51, 52, 53, 54 are formed of an annular member. As shown in FIG. 4, the bending pieces 51, 52, 53, 54 are disposed by being coaxially arranged in a line in the long axis direction of the insertion portion 42. The bending pieces 51, 52, 53, 54 are sequentially called a first bending piece 51, a second bending piece 52, a third bending piece 53, and a fourth bending piece 54 from the extreme end side thereof.

The first and second bending pieces 51, 52 are rotatably connected to each other around a first rotation shaft 61 and rotatably coupled with each other by the first rotation shaft 61. The axial direction of the first rotation shaft 61 is orthogonal to the long axis direction of the insertion portion 42 and the first rotation shaft 61 is disposed in a direction along the up and down directions shown in FIG. 4. Accordingly, the first and second bending pieces 51, 52 can be relatively rotated in right and left directions when viewed from the proximal end (base end) side in FIG. 4.

The second and third bending pieces 52, 53 are rotatably connected to each other around a second rotation shaft 62 and rotatably coupled with each other by the second rotation shaft 62. The axial direction of the second rotation shaft 62 is orthogonal to the long axis direction of the insertion portion 42 and the second rotation shaft 62 is disposed in a direction along the right and left directions shown in FIG. 4. Accordingly, the second and third bending pieces 52, 53 can be relatively rotated in the up and down directions when viewed from the proximal end (base end) side in FIG. 4.

The third and fourth bending pieces 53, 54 are rotatably connected to each other around a third rotation shaft 63 and rotatably coupled with each other by the third rotation shaft 63. The axial direction of the third rotation shaft 63 is orthogonal to the long axis direction of the insertion portion 42 and the third rotation shaft 63 is disposed in the direction along the up and down directions shown in FIG. 4. Accordingly, the third and fourth bending pieces 53, 54 can be relatively rotated in the right and left directions when viewed from the proximal end (base end) side in FIG. 4.

That is, the first rotation shaft 61 constitutes a joint for relatively rotating the first and second bending pieces 51, 52 in the right and left directions. The second rotation shaft 62 constitutes a joint for relatively rotating the second and third bending pieces 52, 53 in the up and down directions. The third rotation shaft 63 constitutes a joint for relatively rotating the third and fourth bending pieces 53, 54 in the right and left directions.

In the embodiment, the axial directions of the first, second, and third rotation shafts 61, 62, 63 are alternately offset by 90°. That is, the bending pieces 51, 52 and the bending pieces 53, 54 are rotated in the right and left directions. The bending pieces 52, 53 are rotated in the up and down directions. Further, the axial directions of the rotation shafts 61, 62, 63 are orthogonal to the center axis (long axis) L of the bending portion 46 (refer to FIGS. 4, 5A and 5B). The center axis L agrees with the long axis of the insertion portion 42.

As shown in FIGS. 5A and 5B, the bending pieces 51, 52, 53, 54 have tongue-piece-shaped coupling portions 65 projecting from the end edges thereof. When the coupling portions 65 are overlapped with each other, the rotation shafts 61, 62, 63 pass through the overlapping portions. That is, the rotation shafts 61, 62, 63 are rivet-like shaft members.

The multijointed bending mechanism arranged as described above is covered with a flexible casing (not shown). The bending portion 46 is constructed by this configuration.

A first set of a pair of non-expandable manipulation wires 56 (56a, 56b) connected to the first bending piece 51, a second set of a pair of non-expandable manipulation wires 57 (57a, 57b) connected to the second bending piece 52, and a third set of a pair of non-expandable manipulation wires 58 (58a, 58b) connected to the third bending piece 53 are inserted into the insertion portion 42.

As shown in FIG. 5A, the manipulation wires 56a, 56b are laterally symmetrically disposed in the bending portion 46 with respect to the center axis L. The extreme ends of the manipulation wires 56a, 56b extend to the region in the first bending piece 51 and are connected to the first bending piece 51.

The direction of the center axis of the first bending piece 51 approximately agrees with the direction of the center axis L. On one plane which passes through both the direction of the center axis of the first bending piece 51 and the axial direction of the first rotation shaft 61, the right half portion of the first bending piece 51 is called a right portion, and the left half portion of the first bending piece 51 is called a left portion.

The extreme end of the manipulation wire 56a described above is connected to the right portion of the first bending piece 51. Further, the extreme end of the manipulation wire 56b is connected to the left portion of the first bending piece 51. When the manipulation wire 56a is pulled to the base end (proximal end) side shown in FIG. 5A, the first bending piece 51 is rotated rightward around the first rotation shaft 61. Further, when the manipulation wire 56b is pulled to the base end side, the first bending piece 51 is rotated leftward around the first rotation shaft 61. As described above, the manipulation wires 56 rotate the first bending piece 51.

As shown in FIG. 5B, the manipulation wires 57a, 57b are vertically symmetrically disposed in the bending portion 46 with respect to the center axis L. The extreme ends of the manipulation wires 57a, 57b extend to the region in the second bending piece 52 and are connected to the second bending piece 52.

The direction of the center axis of the second bending piece 52 approximately agrees with the direction of the center axis L. On one plane which passes through both the direction of the center axis of the second bending piece 52 and the axial direction of the second rotation shaft 62, the upper half portion of the second bending piece 52 is called an upper portion, and the lower half portion of the second bending piece 52 is called a lower portion.

The extreme end of the manipulation wire 57a described above is connected to the upper portion of the second bending piece 52. Further, the extreme end of the manipulation wire 57b is connected to the lower portion of the second bending piece 52. When the manipulation wire 57a is pulled to the base end (proximal end) side shown in FIG. 5B, the second bending piece 52 is rotated upward around the second rotation shaft 62. Further, when the manipulation wire 57b is pulled to the base end side shown in FIG. 5B, the second bending piece 52 is rotated downward around the second rotation shaft 62. As described above, the manipulation wires 57 rotate the second bending piece 52.

As shown in FIG. 5A, the manipulation wires 58a, 58b are laterally symmetrically disposed in the bending portion 46 with respect to the center axis L. The extreme ends of the manipulation wires 58a, 58b extend to the region in the third bending piece 53 and are connected to the third bending piece 53.

The direction of the center axis of the third bending piece 53 approximately agrees with the direction of the center axis L. On one plane which passes through both the direction of the center axis of the third bending piece 53 and the axial direction of the third rotation shaft 63, the right half portion of the third bending piece 53 is called a right portion, and the left half portion of the third bending piece 53 is called a left portion.

The extreme end of the manipulation wire 58a described above is connected to the right portion of the third bending piece 53. Further, the extreme end of the manipulation wire 58b is connected to the left portion of the third bending piece 53. When the manipulation wire 58a is pulled to the base end (proximal end) side shown in FIG. 5A, the third bending piece 53 is rotated rightward around the third rotation shaft 63. Further, when the manipulation wire 58b is pulled to the base end side shown in FIG. 5A, the third bending piece 53 is rotated leftward around the third rotation shaft 63. As described above, the manipulation wires 58 rotate the third bending piece 53.

As described above, the pairs of the manipulation wires 56, 57, 58, which individually correspond to each other, are connected to the bending pieces 51, 52, 53. When the pairs of the manipulation wires 56, 57, 58 are appropriately selected and pushed and pulled in the bending portion 46, the bending pieces 51, 52, 53 are independently rotated.

Various methods can be employed to connect the extreme ends of the manipulation wires 56, 57, 58 to the bending pieces 51, 52, 53. The connection is made as described below in the embodiment.

As shown in FIG. 5A, in the base end portion of the first bending piece 51, cut and raised pieces 70, which project inside of the first bending piece 51, are formed in the right portion and the left portion of the first bending piece 51. The extreme end of the manipulation wire 56a is inserted into the cut and raised piece 70 in the right portion, and fixed to the cut and raised piece 70 by brazing. Further, the extreme end of the manipulation wire 56b is inserted into the cut and raised piece 70 in the left portion, and fixed to the cut and raised piece 70 by brazing.

As shown in FIG. 5B, in the base end portion of the second bending piece 52, cut and raised pieces 70, which project inside of the second bending piece 52, are formed in the upper portion and the lower portion of the second bending piece 52. The extreme end of the manipulation wire 57a is inserted into the cut and raised piece 70 in the upper portion, and fixed to the cut and raised piece 70 by brazing. Further, the extreme end of the manipulation wire 57b is inserted into the cut and raised piece 70 in the lower portion, and fixed to the cut and raised piece 70 by brazing.

As shown in FIG. 5A, in the periphery of the base end portion of the third bending piece 53, cut and raised pieces 70, which project inside of the third bending piece 53, are formed in the right portion and the left portion of the third bending piece 53. The extreme end of the manipulation wire 58a is inserted into the cut and raised piece 70 in the right portion, and fixed to the cut and raised piece 70 by brazing. Further, the extreme end of the manipulation wire 58b is inserted into the cut and raised piece 70 in the left portion, and fixed to the cut and raised piece 70 by brazing.

The manipulation wires 56 are inserted into a guide sheath 66, the manipulation wires 57 are inserted into a guide sheath 67, and the manipulation wires 58 are inserted into a guide sheath 68, and they are individually guided up to the manipulation unit 41. The guide sheaths 66, 67, 68 have flexibility and are formed of a sheath-like elastic member having elasticity; for example, an intimately wound coil, a resin tube, and the like. The inner holes of the guide sheaths 66, 67, 68 are guide members for guiding the direction of travel of the manipulation wires 56, 57, 58.

The extreme end of each guide sheath is not connected to a bending piece to which the manipulation wire to be guided by the guide sheath itself is connected but connected to a bending piece disposed nearer to the base end side than the above bending piece. For example, the extreme ends of guide sheaths 66a, 66b are connected to the second bending piece 52. The extreme ends of guide sheaths 67a, 67b are connected to the third bending piece 53. Further, the extreme ends of guide sheaths 68a, 68b are connected to the fourth bending piece 54.

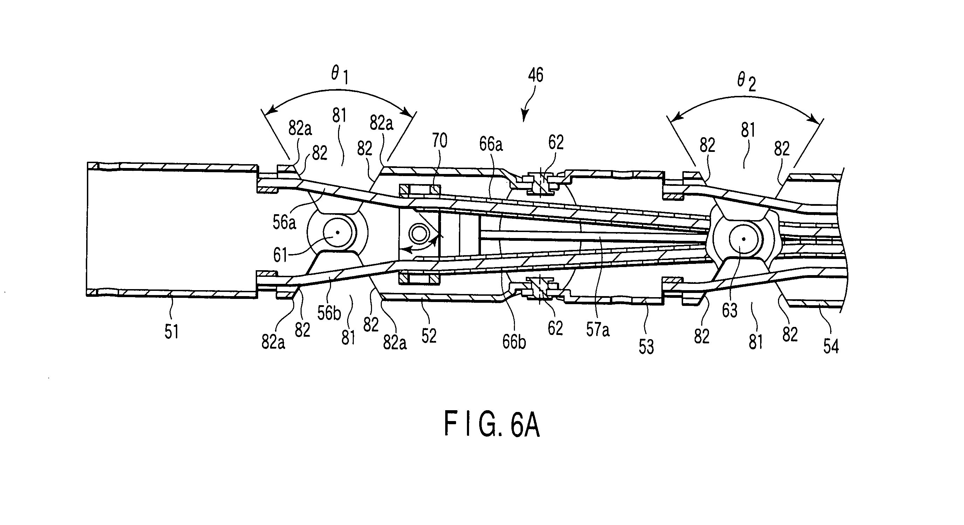

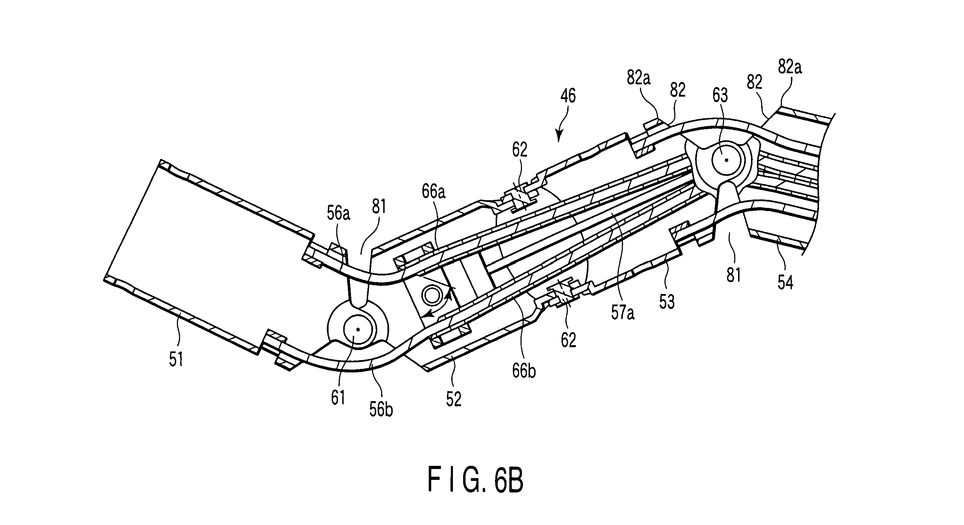

Note that base ends of the guide sheaths may be connected to the base end portion of the bending portion 46 (the extreme end of the flexible tube 45). Further, as shown in FIGS. 6A and 6B, the most extreme end faces of the guide sheaths 66a, 66b may have slant surfaces whose sides near the center of the bending portion 46 retreat to the base end side than the side thereof near the outer periphery of the bending portion 46. As described above, the guide sheaths 66a, 66b may be arranged so that they avoid interference with contained members.

Next, the angular relationship under which the respective bending pieces mutually rotate will be explained with reference to FIGS. 6A and 6B.

The end faces 82, which confront each other (which are adjacent to each other) in adjacent bending pieces, form a gap 81. The gap 81 expands in a fan-shape at an angle θ around the axis of a rotation shaft. In more detail, lines extending from the end faces 82 intersect on the axis of the rotation shaft. Accordingly, the respective end faces 82 are formed as linear end edges passing through the rotation axis, respectively. Then, the gap 81 is formed by the two end faces 82 confronting each other and expanding in a fan-shape at an angle θ about an intersecting point (axis of the rotation shaft).

Note that the extended lines need not necessarily intersect on the axis of the rotation shaft, and the respective end faces 82 may not be formed as linear end edges passing through the rotation axis, respectively. In this case, the gap 81, which expands in a fan-shape at the angle θ, may be preferably formed by the lines which pass through ends (apexes) 82a, which are positioned at the most external sides of the end faces 82, and the axis of the rotation shaft.

Note that the sum of the angles θ of the gaps 81 of at least two adjacent bending pieces in the bending pieces rotating in the same direction is set to 90° or more. As shown in, for example, FIGS. 6A and 6B, the sum of the rotatable angle θ1 of the gap 81 between the bending pieces 51, 52 rotating in the same direction and the rotatable angle θ2 of the gap 81 between the bending pieces 53, 54 rotating in the same direction is set to 90° or more.

As described above, the rotatable angle θ of the multijointed bending piece may be preferably allocated not only to one gap 81 but also to the gaps 81 between the bending pieces rotating in the same direction (a plurality of adjacent gaps 81). With this configuration, it is not necessary to increase the angle θ in one gap 81. Accordingly, the maximum angle θ formed by one gap 81 is reduced. As a result, the amount of rotation of a bending piece in one gap 81 is reduced. Thus, when a bending operation causes the contained members such as the manipulation wires and the guide sheaths to traverse the gap 81, they are less likely to be caught by the gap 81.

As shown in FIG. 4, the fourth bending piece 54 is a bending piece positioned at the most extreme base end of the bending portion 46. That is, it is possible to assume that the fourth bending piece 54 is the base end portion of the bending portion 46. A connector member 83 such as a connection ring is disposed in the extreme end of the flexible tube 45. The fourth bending piece 54 is coupled with the connector member 83. Further, the fourth bending piece 54 may be rotatably coupled with the connector member 83. In this mode, it is also possible to assume that the connector member 83 is the base end portion of the bending portion 46.

Note that although the fourth bending piece 54 is connected and fixed to the connector member 83, the fourth bending piece 54 need not be limited thereto. When, for example, the number of the bending pieces is increased, a fifth bending piece (not shown) is rotatably connected to the fourth bending piece 54 and further a sixth bending piece (not shown) is rotatably connected to the fifth bending piece so that they are coupled with each other. The sixth bending piece is connected to the connector member 83.

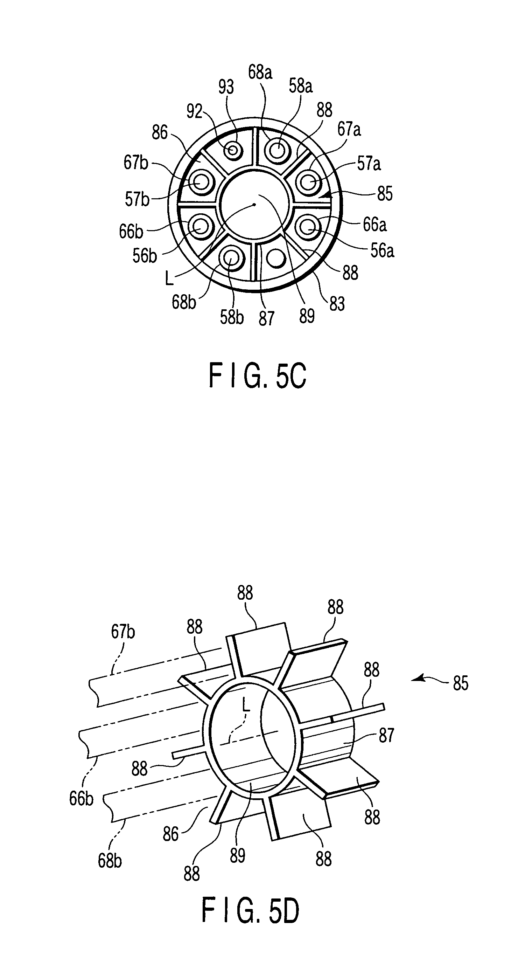

Next, disposition of the manipulation wires and the guide sheaths in the bending pieces and positioning/disposing means will be explained with reference to FIGS. 5C and 5D.

As shown in FIGS. 5A and 5B, a frame-like separator 85 shown in FIGS. 5C and 5D is disposed in the connector member 83. The separator 85 is the positioning/disposing means for determining the positions of the manipulation wires and the guide sheaths into which the manipulation wires are inserted. The guide sheaths for guiding the manipulation wires are concentrically disposed around the inner periphery of the bending portion 46 by the separator 85.

Accordingly, the manipulation wires 56, 57, 58 are disposed concentrically in a direction vertical to the axial directions of the first rotating shaft 61, the second rotating shaft 62, and the third rotating shaft 63 (the direction of the center axis L).

In other words, the separator 85 executes positioning so that the manipulation wires 56, 57, 58 are concentrically disposed. The separator 85 may be disposed in the fourth bending piece 54.

As shown in FIG. 5D, the separator 85 has a cylindrical portion 87. A plurality of partition plates 88 are disposed in the outer periphery of the cylindrical portion 87 at approximately the same intervals along the center axis L. The center axis of the cylindrical portion 87 agrees with the center axis L. Further, the partition plates 88 are approximately radially disposed in the center axis of the cylindrical portion 87. Accordingly, a plurality of accommodation paths 86 are formed between the partition plates 88 in the peripheral direction of the cylindrical portion 87. That is, the accommodation paths 86 are concentrically disposed along the peripheral direction of the cylindrical portion 87 around the cylindrical portion 87. The accommodation paths 86 cause the manipulation wires 56, 57, 58 to pass therethrough.

As described above, the separator 85 forms the accommodation paths 86, through which the manipulation wires 56, 57, 58 pass, by the partition plates 88. The separator 85 concentrically positions the manipulation wires 56, 57, 58 by causing them to pass through the accommodation paths 86.

Note that an inner hole of the cylindrical portion 87 is used as an insertion path 89 for inserting contained members and the like thereinto. The insertion path 89 is a space in which other contained members are disposed and positioned in a central region of the insertion portion 42.

As described above, the manipulation wires and the guide sheaths are concentrically positioned and disposed in peripheral portions in the bending portion 46 and the insertion portion 42 by the separator 85.

Further, as shown in FIG. 5C, each sets of the manipulation wires and the guide sheaths are separately disposed in the accommodation paths 86. Note that different sets of the manipulation wires and the guide sheaths may be disposed in one accommodation path 86. The manipulation wires and the guide sheaths are guided from the separator 85 up to the manipulation unit 41 through the insertion portion 42.

Further, when the number of the accommodation paths 86 is larger than that of the guide sheaths, the other contained members (for example, a manipulation wire 92 for manipulating the grip forceps 48, a signal line, and the like) are disposed in the accommodation path 86 in which no guide sheath is disposed as shown in FIG. 5C. The manipulation wire 92 is inserted into a guide sheath 93 having flexibility and guided up to the manipulation unit 41 through the flexible tube 45.

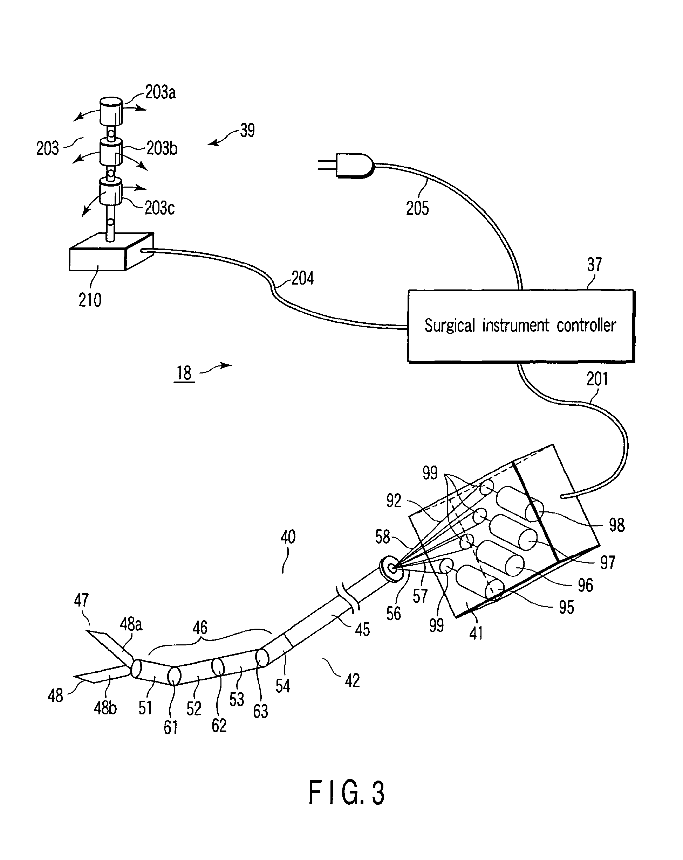

As shown in FIG. 3, the manipulation unit 41 is provided with a bending portion manipulation mechanism and a surgical portion manipulation mechanism. The bending portion manipulation mechanism is provided with drive motors 95, 96, 97 for pushing and pulling the manipulation wires 56, 57, 58, respectively. Further, the surgical portion manipulation mechanism is provided with a drive motor 98 for pushing and pulling the manipulation wire 92. The manipulation wires 56, 57, 58 correspond to the bending pieces (targets to be rotated) 51, 52, 53 and execute rotating manipulations. The manipulation wire 92 manipulates the grip forceps 48.

Pulleys 99 are attached to drive shafts of the drive motors 95, 96, 97, 98, respectively. The respective drive shafts may be coupled with the respective pulleys 99 through reducers (not shown). The manipulation wires 56, 57, 58, 92 are trained round the respective pulleys 99. The drive motors 95, 96, 97, 98 are individually driven, respectively, and when the pulleys 99 are rotated, the manipulation wires 56, 57, 58, 92 trained around the pulleys 99 are pushed and pulled.

Although the bending portion manipulation mechanism and the surgical portion manipulation mechanism use transmission mechanisms making use of the pulleys 99, they may use, for example, a gear mechanism and the like making use of a pinion gear and a rack. Further, the bending portion manipulation mechanism and the surgical portion manipulation mechanism may use other types of drive actuators in place of the drive motors 95, 96, 97, 98.

As shown in FIGS. 2 and 3, the manipulation unit 41 is connected to the surgical instrument controller 37 through a cable 201. The bending manipulation unit 39 as the manipulation input unit is connected to the surgical instrument controller 37 through a cable 204. In FIG. 3, the surgical instrument controller 37 is provided with a power supply cord 205.

The bending manipulation unit 39 includes a joystick (manipulation input unit) 203 for instructing a position and an attitude of the surgical instrument 40. The joystick 203 includes three joystick switches 203a, 203b, 203c continuously connected in three stages. The joystick switches 203a, 203b, 203c are attached to a manipulation box 210.

When the joystick switches 203a, 203b, 203c are selectively manipulated, the drive motors 95, 96, 97 are individually driven corresponding to the manipulation. With this manipulation, the bending pieces 51, 52, 53 are individually and independently driven in up, down, right, and left directions to thereby bend respective joint portions.

The surgical instrument extreme end movement controller 18 can move the extreme end portion 47 to a desired position by the movement according to the manipulation of the joystick 203. That is, the surgical instrument extreme end movement controller 18 constitutes the surgical instrument 40 which is arranged as a master/slave type and driven electrically. Note that, when the joystick 203 is manipulated by an operator and the like after a control for moving the surgical instrument 40 is set, preference is given to an instruction for manipulating the joystick 203.

As shown in FIG. 2, the surgical instrument controller 37 is provided with a function control input portion 121 for inputting an instruction output from the joystick 203, a condition for controlling the function of the joystick 203, and the like, a motor driver (surgical instrument drive controller) 122 for controlling the drive of the drive motors 95, 96, 97, and a motor unit communication unit 123 connected to the surgical instrument drive unit 38 through the cable 201 for executing communication with the surgical instrument drive unit 38.

The surgical instrument controller 37 transmits a control signal for driving the drive motors 95, 96, 97 in response to the manipulation of the joystick 203 executed by the operator to the motor driver 122 and rotates the drive motors 95, 96, 97. Encoders (not shown) are mounted on the drive motors 95, 96, 97 to measure the number of revolutions thereof. The encoders feedback-control the drive motors 95, 96, 97 by generating signals according to the number of revolutions and transmitting the signals to the motor driver 122.

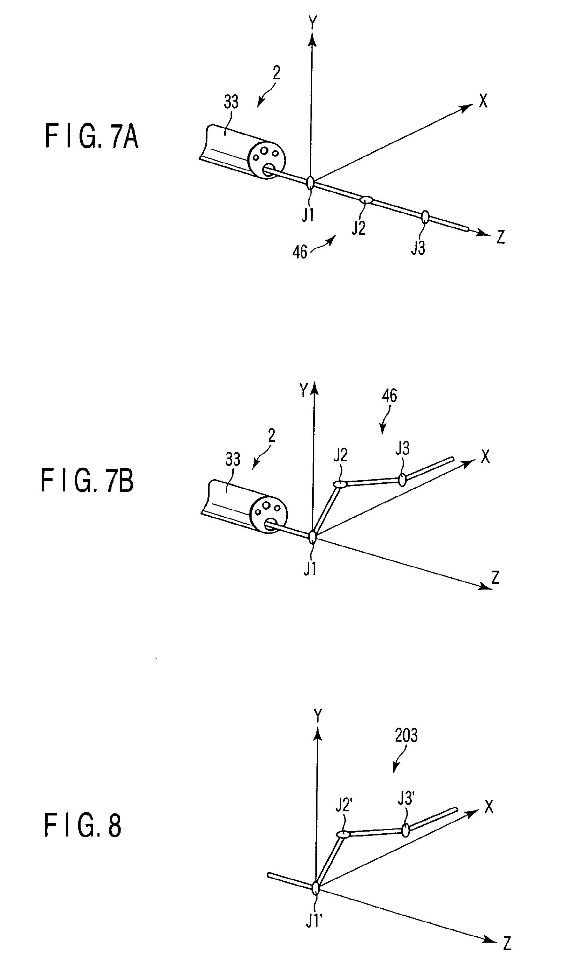

The relation between a multijointed structure in the bending portion 46 and the joystick 203 will be explained with reference to FIGS. 7A, 7B and 8.

As shown in FIG. 7A, in a state that all the joint portions in the bending portion 46 project from the extreme end portion 33, the joints disposed from the manipulation unit side (base end side) to the extreme end side are sequentially referred to as J1, J2, J3. A coordinate system is set using the joint J1 disposed nearest to the manipulation unit side as a reference. In the coordinate system, a Y-axis direction agrees with a vertical direction of the image pickup device. It is assumed that the joints J1, J3 are bent about an X-axis, and the joint J2 is bent about a Y-axis.

As shown in FIG. 8, the joystick 203 (manipulation input unit) includes joints J1', J2', J3' which have the same structures as those of the joint J1 and the joints J2, J3 located nearer to the extreme end side than the joint J1.

The number of the joints and the bending directions of the joystick 203 are the same as those of the bending portion 46. The lengths of respective rods of the joystick 203 are set to values multiplied by an appropriate coefficient k so that the operator can easily manipulate them. When, for example, k = 10 and the length of each rod of the surgical instrument 40 is 3 mm, the length of each rod of the joystick 203 (manipulation input unit) is set to 30 mm. Encoders (not shown) are assembled to the joints J1', J2', J3' to measure bent angles. The information of the bent angles measured by the encoders is sent to the surgical instrument controller 37. The surgical instrument controller 37 generates drive signals corresponding to the angle information (the joints J1', J2', J3') and bends the joints J1, J2, J3 by rotating the drive motors 95, 96, 97, respectively. When the joints J1', J2', J3' are bent as shown in, for example, FIG. 8, the joints J1, 2, J3 are bent as shown in FIG. 7B.

Since the bending portion 46 has a plurality of joints, the extreme end of the surgical instrument 40 can be moved to an arbitrary position and an arbitrary attitude so that an affected area can be more easily cut out and exfoliated than ever before. Further, since the joint structure of the surgical instrument 40 is caused to equally correspond to that of the manipulation input unit, the operator can easily operate the surgical instrument having a plurality of joints.

Further, the drive motor 98 also has a motor driver, a motor unit communication unit, and the like similarly to the drive motors 95, 96, 97. The grip forceps 48 is manipulated by manipulating a manipulation body such as a handle (function control/input unit) 125 disposed in the manipulation unit 41 and the like.

Note that the manipulation input unit may be preferably provided with a first manipulation switch corresponding to the first bending piece 51, a second manipulation switch corresponding to the second bending piece 52, a third manipulation switch corresponding to the third bending piece 53, and a fourth manipulation switch corresponding to the fourth bending piece 54. When, for example, the first manipulation switch is depressed, the first bending piece 51 is bent. Further, the manipulation unit 41 may be preferably provided with a switch device (manipulation switch) for the bending manipulation. The manipulation input unit may use a pen type input unit for inputting a three-dimensional position.

Next, an operation when the surgical instrument 40 is used will be explained.

First, as shown in FIG. 2, the insertion portion 21 is inserted into a body cavity, and the insertion portion 42 is inserted from the insertion port 28 into the insertion channel in this state. The extreme end portion 47 and the bending portion 46 project from the channel port 36 into the body cavity. Then, a work for gripping an affected area and the like in the body cavity is executed using the grip forceps 48 while observing them by the endoscope 2.

In this case, the bending portion 46 can be bent to an appropriate multijointed bent shape according to the state in the body cavity and the surgical procedure. That is, when the joystick 203 is manipulated and the bending pieces 51, 52, 53 are individually rotated, the bending portion 46 is bent into an appropriate shape.

When, for example, the drive motor 95 is driven, the manipulating wires 56a, 56b trained around the pulley 99 in the drive motor 95 are pushed and pulled. With this operation, the first bending piece 51 is independently rotated. When the drive motor 96 is driven, the manipulation wires 57a, 57b trained around the pulley 99 in the drive motor 96 are pushed and pulled. With this operation, the second bending piece 52 is independently rotated. Further, when the drive motor 97 is driven, the manipulation wires 58a, 58b trained around a pulley 99 in the drive motor 97 are pushed and pulled. With this operation, the third bending piece 53 is independently rotated.

Accordingly, when the joystick 203 is appropriately operated, the bending pieces 51, 52, 53 are independently rotated, and the bending portion 46 is bent. The bending portion 46 can even be bend in a complex shape by adjusting the direction in which the joystick 203 is rotated and the amount of rotation thereof.

As described above, in the embodiment, since the manipulation wires are disposed in the plurality of bending pieces, only an arbitrary bending piece can be independently rotated. Accordingly, in the embodiment, since the bending mechanism has a plurality of degrees of freedom, a work can be executed even in a narrow region such as a body cavity.

In more detail, in the embodiment, since the bending pieces 51, 52, 53 can be independently rotated (bent), the bending portion 46 can be partially bent also in a different direction. Thus, in the embodiment, the bending portion 46 can be bent into an appropriate shape according to a state of use. As a result, since the degree of freedom of bending of the bending portion 46 is increased, it is possible in the embodiment to easily execute even a complex work in a narrow body cavity region as compared with a case in which the bending portion 46 is uniformly bent. Further, in the embodiment, since the attitude of the bending portion 46 can be easily bent so that it does not disturb another surgical instrument or observation with the endoscope 2, the workability of the surgical instrument 40 can be increased.

Further, in the embodiment, since the plurality of wires are concentrically disposed in the bending pieces, the plurality of manipulation wires can be disposed compactly in the bending pieces. Accordingly, the embodiment can avoid complicated disposition in which the plurality of manipulation wires are entangled with each other. Further, the embodiment can form the space in the bending pieces in which the other contained members are disposed.

To explain in detail, in the embodiment, the manipulation wires 56, 57, 58 and the guide sheaths 66, 67, 68 are inserted into the accommodation paths 86 and concentrically disposed therein. Accordingly, in the embodiment, the manipulation wires 56, 57, 58 can be disposed without being entangled with each other even though they are inserted into the narrow bending portion 46, and further the manipulation wires 56, 57, 58 can be disposed compactly. In other words, in the embodiment, since the manipulation wires 56, 57, 58 can be prevented from being entangled in the bending portion 46, occurrence of mutual interference of the manipulation wires can be reduced. In the embodiment, disturbance of transmission of the manipulation force of the manipulation wires 56, 57, 58 can be prevented. Since the embodiment can obtain an allowance for disposing the other contained member in the bending portion 46, the diameter of the bending portion 46 can be reduced.

Further, in the embodiment, the manipulation wires 56, 57, 58 are concentrically disposed. Accordingly, the embodiment can form the insertion path 89 as the space in the bending pieces in which the other contained members are installed.

Further, in the embodiment, a guide sheath, which guides a manipulation wire for rotating a bending piece, is connected to a bending piece located just behind the above bending piece (on the base end side). Thus, the embodiment can maximize the efficacy of the wire guide function achieved by the guide sheath. Further, the region in which the manipulation wires are separately exposed can be reduced. Accordingly, the embodiment can avoid any reduction in the wire guide functionality. Further, when, for example, the insertion portion 42 itself is twisted, the embodiment can alleviate the effect of the twist on the wire guide function of the guide sheaths.

Further, in the embodiment, the guide sheaths may be formed of an intimately wound metal coil. With this configuration, the embodiment can sufficiently withstand the abrupt rotating and bending actions of the bending pieces.

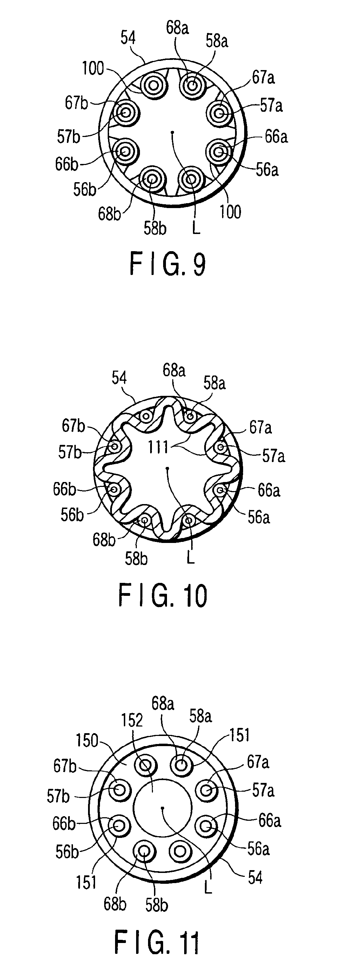

Other modification of the positioning/disposing means (separator) in the embodiment described above will be explained with reference to FIG. 9.

Respective separators 100 as positioning/disposing means in the modification are concentrically disposed on a circle about the center axis L at the same intervals around the inner periphery of the fourth bending piece 54. The separators 100 are fixed to the inner periphery of the fourth bending piece 54 by brazing. Further, the separators 100 are composed of a metal pipe member.

Note that, although the respective separators 100 are disposed at the same intervals, they need not necessarily be disposed at the same intervals.

Then, the guide sheaths 66, 67, 68 are inserted into the holes of the respective separators 100 so as to freely move forward and rearward therethrough. With this configuration, the respective separators 100 position the guide sheaths 66, 67, 68. Accordingly, the guide sheaths 66, 67, 68 are concentrically disposed.

As described above, in the modification, since the pipe-like separators 100 are used as the positioning/disposing means, the manipulation wires 56, 57, 58 can be disposed more compactly than the frame-like separator 85 in the embodiment described above.

Note that the separator 100 may be disposed in the connector member 83.

Other modification of the positioning/disposing means (piece portion) in the embodiment described above will be explained with reference to FIG. 10.

Positioning/disposing means in the modification has a plurality of flange- or rib-shaped piece portions 111 in the fourth bending piece 54. The piece portions 111 are formed by being cut and raised to a U-shape toward the inside of the fourth bending piece 54. The piece portions 111 are disposed on a circle about the center axis L at the same intervals.

Then, the guide sheaths 66, 67, 68 are inserted into the piece portions 111 so as to freely move forward and rearward therethrough. With this configuration, the piece portions 111 position the guide sheaths 66, 67, 68. Accordingly, the guide sheaths 66, 67, 68 are concentrically disposed.

As described above, since the modification uses the piece portions 111 as the positioning/disposing means, it can reduce the number of parts as compared with the separators 85, 100.

Note that the piece portions 111 may be formed in the connector member 83.

Next, other modification of the positioning/disposing means (disc) in the embodiment described above will be explained with reference to FIG. 11.

Positioning/disposing means in the modification has a disc 150 in, for example, the fourth bending piece 54. In, for example, the fourth bending piece 54, the disc 150 is disposed at right angles and coaxially to the center axis L. A plurality of holes 151 are formed in the disc 150 on a coaxial circle about the center axis L.

The guide sheaths 66, 67, 68 are inserted into the holes 151 so as to freely move forward and rearward therethrough. With this configuration, the disc 150 positions the guide sheaths 66, 67, 68. Accordingly, the guide sheaths 66, 67, 68 are concentrically disposed.

Further, a through hole 152 is formed at the center of the disc 150. The through hole 152 is used as a space into which the other contained members are inserted.

Note that, in the modification, the positioning/disposing means may have the disc 150 in, for example, the connector member 83.

Although the guide sheaths 66, 67, 68 are single-concentrically disposed in the embodiment and the modifications described above, they may be double-concentrically disposed. That is, a plurality of manipulation wires (a first group) disposed concentrically and a plurality of manipulation wires (a second group) disposed concentrically and externally of the first group may be positioned. As described above, when the manipulation wires are positioned double-concentrically in the embodiment and the modifications described above, many manipulation wires can be disposed compactly.

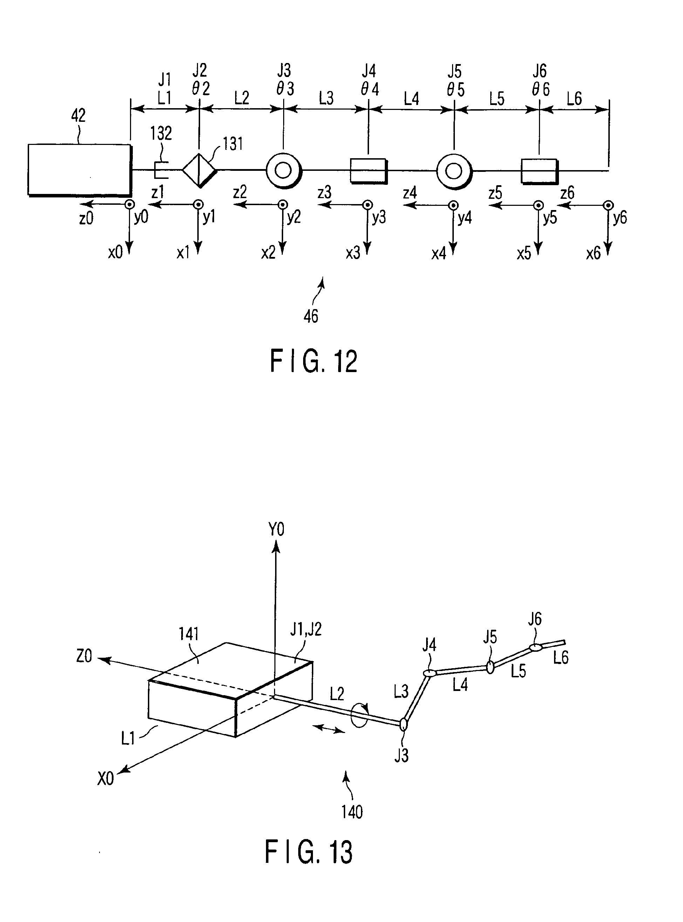

Next, another embodiment of the present invention will be explained, with reference to FIGS. 12 and 13. The overall configuration of an endoscope apparatus system in this embodiment is approximately the same as that of the above-described embodiment. However, a motor unit of a surgical instrument 40 is additionally provided with a mechanism 131 for rotating a bending portion 46 around the axis of an insertion portion 42 and a mechanism 132 for advancing the bending portion 46 in the axial direction of the insertion portion 42 in parallel therewith. Further, at least four joints are disposed in the bending portion 46. With this configuration, the position and the attitude of the extreme end portion 47 are arbitrarily controlled. Further, the movement of the surgical instrument 40 corresponds to that of a manipulation input unit 140. A joystick type manipulation input unit having an advancing, retreating, and rotating joint structure is used as the manipulation input unit 140.

A coordinate system is set as shown in FIG. 12. The coordinate system uses a base end portion 141 of the manipulation input unit 140 as a reference and corresponds to the surgical instrument 40. In the coordinate system, the joint J1 moves forward and rearward, the joint J2 rotates in an axial direction, the joints J3, J5 are bent about a Y-axis, and the joints J4, J6 are bent about an X-axis. The rotation angles of the joints J2 to J6 are shown by θ2 to θ6, respectively. The lengths of respective rods are shown by L1 to L5 and the length of an extreme end rod is shown by L6. Thus, conversion matrices in the respective joints J1, J2, J3, J4, J5, J6 are shown by Expression 1 from the kinematics of the manipulator (surgical instrument 40).

[Expression 1]

$$\mathrm{Joint\,J1:}\ T_0^1 = \begin{pmatrix} 1 & 0 & 0 & 0 \\ 0 & 1 & 0 & 0 \\ 0 & 0 & 1 & -L_1 \\ 0 & 0 & 0 & 1 \end{pmatrix}$$

$$\mathrm{Joint\,J2:}\ T_1^2 = \begin{pmatrix} \cos\theta_2 & -\sin\theta_2 & 0 & 0 \\ \sin\theta_2 & \cos\theta_2 & 0 & 0 \\ 0 & 0 & 1 & -L_2 \\ 0 & 0 & 0 & 1 \end{pmatrix}$$

$$\mathrm{Joint\,J3:}\ T_2^3 = \begin{pmatrix} \cos\theta_3 & 0 & \sin\theta_3 & -L_3\sin\theta_3 \\ 0 & 1 & 0 & 0 \\ -\sin\theta_3 & 0 & \cos\theta_3 & -L_3\cos\theta_3 \\ 0 & 0 & 0 & 1 \end{pmatrix}$$

$$\mathrm{Joint\,J4:}\ T_3^4 = \begin{pmatrix} 1 & 0 & 0 & 0 \\ 0 & \cos\theta_4 & -\sin\theta_4 & L_4\sin\theta_4 \\ 0 & \sin\theta_4 & \cos\theta_4 & -L_4\cos\theta_4 \\ 0 & 0 & 0 & 1 \end{pmatrix}$$

$$\mathrm{Joint\,J5:}\ T_4^5 = \begin{pmatrix} \cos\theta_5 & 0 & \sin\theta_5 & -L_5\sin\theta_5 \\ 0 & 1 & 0 & 0 \\ -\sin\theta_5 & 0 & \cos\theta_5 & -L_5\cos\theta_5 \\ 0 & 0 & 0 & 1 \end{pmatrix}$$

$$\mathrm{Joint\,J6:}\ T_5^6 = \begin{pmatrix} 1 & 0 & 0 & 0 \\ 0 & \cos\theta_6 & -\sin\theta_6 & L_6\sin\theta_6 \\ 0 & \sin\theta_6 & \cos\theta_6 & -L_6\cos\theta_6 \\ 0 & 0 & 0 & 1 \end{pmatrix}$$

Accordingly, a homogeneous conversion matrix is shown by Expression 2.

[Expression 2]

$$T_0^6 = T_0^1 T_1^2 T_2^3 T_3^4 T_4^5 T_5^6$$

$$= \begin{pmatrix} r_{11} & r_{12} & r_{13} & t_x \\ r_{21} & r_{22} & r_{23} & t_y \\ r_{31} & r_{32} & r_{33} & t_z \\ 0 & 0 & 0 & 1 \end{pmatrix}$$

Since the coordinate system uses the base end portion 141 as the reference, the position (x, y, z) and the attitude ($\theta$x, $\theta$y, $\theta$z) of the extreme end portion of the manipulation input unit 140 are determined by Expression 3.

[Expression 3]

$$\begin{pmatrix} x \\ y \\ z \end{pmatrix}^T = \begin{pmatrix} t_x \\ t_y \\ t_z \end{pmatrix}^T \qquad \begin{pmatrix} \theta_x \\ \theta_y \\ \theta_z \end{pmatrix}^T = \begin{pmatrix} \mathrm{asin}(r_{32}/\cos\theta_y) \\ \mathrm{asin}(-r_{31}) \\ \mathrm{asin}(r_{21}/\cos\theta_y) \end{pmatrix}^T$$

The configuration of the surgical instrument 40 is different from that of the manipulation input unit 140. Accordingly, to operate the surgical instrument 40 by operating the manipulation input unit 140, it is necessary to match the position and the attitude of the surgical instrument 40 with those of the manipulation input unit 140. For this purpose, the rotation angles and the amounts of parallel (forward and rearward) movement of the respective joints of the surgical instrument 40 must be determined.

As described above, the movement of the surgical instrument 40 corresponds to that of the manipulation input unit 140. Accordingly, the position and the attitude of the surgical instrument 40 are determined by those of the manipulation input unit 140. Assuming that the configuration of the surgical instrument 40 is known, the rotation angles and the amounts of parallel movement of the respective configurations of the surgical instrument 40 can be determined by inverse kinematics. Inverse kinematics is a method of estimating the specific values of the joints (angles and the like thereof) from the position/attitude information of the manipulator (surgical instrument 40) in a working space. The joint parameter $\Phi$ of the respective joints 1, 2, ... , n are shown by Expression 4.

[Expression 4]

$$\Phi = (\theta_1, \ \theta_2, \ \cdots, \ \theta_n)^T$$

The position and the attitude of the manipulator are shown by Expression 5.

[Expression 5]

$$E_p = (x_{Ep}, \ y_{Ep}, \ z_{Ep}, \ \mathrm{Roll}_{Ep}, \ \mathrm{Yaw}_{Ep}, \ \mathrm{Pitch}_{Ep})^T$$

Thus, the relation thereof is shown by Expression 6.

[Expression 6]

$$E_p = A(\Phi)$$

Here, the target P of the position and the attitude of the manipulator is shown by Expression 7.

[Expression 7]

$$P_p = (x_{Pp}, \ y_{Pp}, \ z_{Pp}, \ Roll_{Pp}, \ Yaw_{Pp}, \ Pitch_{Pp})^T$$

To place the manipulator in a Pp state, $\Phi$ must be determined to satisfy Expression 8.

[Expression 8]

$$P_p = A(\Phi)$$

However, since these expressions are non-linear, ordinarily, Jacobian matrix J($\Phi$) is determined by subjecting Ep to partial differentiation by the factor of $\Phi$ to determine $\Phi$.

[Expression 9]

$$J(\Phi) = \begin{pmatrix} dx_{ep}/d\theta_1 & dx_{ep}/d\theta_2 & \cdots & dx_{ep}/d\theta_n \\ dy_{ep}/d\theta_1 & dy_{ep}/d\theta_2 & \cdots & dy_{ep}/d\theta_n \\ dz_{ep}/d\theta_1 & dz_{ep}/d\theta_2 & \cdots & dz_{ep}/d\theta_n \\ dRoll_{ep}/d\theta_1 & dRoll_{ep}/d\theta_2 & \cdots & dRoll_{ep}/d\theta_n \\ dYaw_{ep}/d\theta_1 & dYaw_{ep}/d\theta_2 & \cdots & dYaw_{ep}/d\theta_n \\ dPitch_{ep}/d\theta_1 & dPitch_{ep}/d\theta_2 & \cdots & dPitch_{ep}/d\theta_n \end{pmatrix}$$

Expression 11 is determined from Expression 10.

[Expression 10]

$$\dot{\Phi} = J(\Phi)^{-1}\dot{E}_p$$

[Expression 11]

$$P_p = A(\Phi)$$

Then, $\Phi$ that satisfies Expression 11 is determined by a convergence calculation.

As a result, according to the embodiment, even when the configuration of the manipulation input unit 140 is different from that of the surgical instrument 40, the extreme end of the surgical instrument 40 can be moved to an arbitrary position and an arbitrary attitude from the position and the attitude of the manipulation input unit 140, and an affected area can be cut out and exfoliated more easily than ever before.

The present invention can be also applied to a bending portion of an endoscope. The present invention can be applied to, for example, the bending mechanism of the bending portion in the insertion portion of the endoscope according to the embodiment described above. Further, the surgical instrument as a target of the present invention also includes a surgical catheter.

<Additional statement>

According to the above explanation, there can be obtained multijointed medical equipment according to the following items or arbitrary combinations of the following items and the items according to the claims.

1. An endoscope surgical instrument including:

an endoscope to observe an affected area in a body cavity;
a surgical instrument to perform surgery on the affected area by passing through an insertion portion of the endoscope; '
at least one bending means disposed in an extreme end of the surgical instrument;
manipulation means for moving the extreme end of the surgical instrument in a direction intended by an operator;
control means for controlling movement of the extreme end of the surgical instrument in response to manipulation of the manipulation means; and
means for operating the bending means of the surgical instrument in response to a control signal from the control means.

2. The endoscope surgical instrument according to item 1, wherein the surgical instrument includes a soft insertion portion and a surgical portion for cutting out and exfoliating an affected area of a living body.
3. The endoscope surgical instrument according to item 1, wherein power for operating the bending means is assembled in the vicinity of the extreme end of the surgical instrument.
4. The endoscope surgical instrument according to item 1, in which power for operating the bending means is disposed in a portion other than the vicinity of the extreme end of the surgical instrument and which includes transmission means for transmitting the power to the bending means.
5. The endoscope surgical instrument according to item 1, including means for moving the extreme end of the surgical instrument forward and rearward.

**Claims**

1.  A multijointed bending mechanism **characterized by** comprising:

a first bending piece;
a second bending piece connected to the first bending piece so as to be rotatable around a first rotation shaft;
a third bending piece connected to the second bending piece so as to be rotatable around a second rotation shaft;
at least two first wires connected to the first bending piece to rotate the first bending piece; and
at least two second wires connected to the second bending piece to rotate the second bending piece,
wherein the first wires and the second wires are disposed concentrically with respect to a vertical direction of the first rotation shaft and the second rotation shaft.

2.  The multijointed bending mechanism according to claim 1, **characterized by** further comprising positioning/disposing means disposed in the third bending piece to execute positioning so that the first wires and the second wires are disposed concentrically.

3.  The multijointed bending mechanism according to claim 1, **characterized by** further comprising:

a first elastic member connected to the second bending piece to guide the first wires; and
a second elastic member connected to the third bending piece to guide the second wires.

14

4. The multijointed bending mechanism according to claim 3, **characterized by** further comprising positioning/disposing means disposed in the third bending piece to execute positioning so that the first wires and the second wires are disposed concentrically.

5. A multijointed medical equipment **characterized by** comprising the multijointed bending mechanism according to claim 1.

6. A multijointed bending mechanism **characterized by** comprising:

a first bending piece;
a second bending piece connected to the first bending piece so as to be rotatable around a first rotation shaft;
a third bending piece connected to the second bending piece so as to be rotatable around a second rotation shaft;
a fourth bending piece connected to the third bending piece so as to be rotatable around a third rotation shaft;
at least two first wires connected to the first bending piece to rotate the first bending piece;
at least two second wires connected to the second bending piece to rotate the second bending piece; and
at least two third wires connected to the third bending piece to rotate the third bending piece,
wherein the first wires, the second wires, and the third wires are concentrically disposed in a vertical direction of the first rotation shaft, the second rotation shaft, and the third rotation shaft.

7. The multijointed bending mechanism according to claim 6, **characterized by** further comprising positioning/disposing means disposed in the fourth bending piece to execute positioning so that the first wires, the second wires, and the third wires are disposed concentrically.

8. The multijointed bending mechanism according to claim 7, **characterized in that** the positioning/disposing means forms accommodation paths by partition plates to cause the first wires, the second wires, and the third wires to pass therethrough and positions the first wires, the second wires, and the third wires concentrically by causing the first wires, the second wires, and the third wires to pass through the accommodation paths.

9. The multijointed bending mechanism according to claim 6, **characterized by** further comprising:

a first elastic member connected to the second bending piece to guide the first wires;
a second elastic member connected to the third bending piece to guide the second wires; and
a third elastic member connected to the fourth bending piece to guide the third wires.

10. The multijointed bending mechanism according to claim 9, **characterized by** further comprising positioning/disposing means disposed in the fourth bending piece to execute positioning so that the first wires, the second wires, and the third wires are disposed concentrically.

11. The multijointed bending mechanism according to claim 10, **characterized in that** the positioning/disposing means forms accommodation paths by partition plates to cause the first wires, the second wires, and the third wires to pass therethrough and positions the first wires, the second wires, and the third wires concentrically by causing the first wires, the second wires, and the third wires to pass through the accommodation paths.

12. A multijointed medical equipment comprising the multijointed bending mechanism according to claim 6.

FIG.1

FIG. 2

FIG. 3

FIG. 4

Up
Left
Down
Right

Center axis L of bending portion

FIG. 5A

FIG. 5B

F I G. 5C

F I G. 5D

FIG. 6A

EP 2 127 591 A1

F I G. 6B

FIG. 7A

FIG. 7B

FIG. 8

FIG. 9

FIG. 10

FIG. 11

FIG. 12

FIG. 13

**INTERNATIONAL SEARCH REPORT**

| International application No. |
| --- |
| PCT/JP2007/074239 |

A. CLASSIFICATION OF SUBJECT MATTER
*A61B1/00*(2006.01)i

According to International Patent Classification (IPC) or to both national classification and IPC

B. FIELDS SEARCHED

Minimum documentation searched (classification system followed by classification symbols)
A61B1/00

Documentation searched other than minimum documentation to the extent that such documents are included in the fields searched
Jitsuyo Shinan Koho       1922-1996   Jitsuyo Shinan Toroku Koho   1996-2008
Kokai Jitsuyo Shinan Koho   1971-2008   Toroku Jitsuyo Shinan Koho   1994-2008

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X<br>Y | JP 2003-220022 A  (Olympus Optical Co., Ltd.),<br>05 August, 2003 (05.08.03),<br>(Family: none) | 1,3,5<br>2,4,9-11 |
| X<br>Y | JP 2002-102152 A  (Olympus Optical Co., Ltd.),<br>09 April, 2002 (09.04.02),<br>(Family: none) | 1,5<br>2 |
| X<br>Y | JP 47-012398 B1  (Olympus Optical Co., Ltd.),<br>17 April, 1972 (17.04.72),<br>(Family: none) | 1,5,6,12<br>2,7-11 |
| Y | JP 62-265612 A  (Circon Corp.),<br>18 November, 1987 (18.11.87),<br>& US 4686963 A | 2,4,7,8,10,<br>11 |

| ☒ | Further documents are listed in the continuation of Box C. | ☐ | See patent family annex. |
| --- | --- | --- | --- |

| * | Special categories of cited documents: | "T" | later document published after the international filing date or priority date and not in conflict with the application but cited to understand the principle or theory underlying the invention |
| "A" | document defining the general state of the art which is not considered    to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>16 January, 2008 (16.01.08) | Date of mailing of the international search report<br>29 January, 2008 (29.01.08) |
| --- | --- |
| Name and mailing address of the ISA/<br>Japanese Patent Office | Authorized officer |
| Facsimile No. | Telephone No. |

Form PCT/ISA/210 (second sheet) (April 2007)

27

**INTERNATIONAL SEARCH REPORT**

| International application No. |
| --- |
| PCT/JP2007/074239 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | JP 4-170930 A  (Fuji Photo Optical Co., Ltd.), 18 June, 1992 (18.06.92), & US 5257618 A | 2,4,7,8,10, 11 |

Form PCT/ISA/210 (continuation of second sheet) (April 2007)

**REFERENCES CITED IN THE DESCRIPTION**

**Patent documents cited in the description**

- JP 2003126024 A
- JP 6105797 A